# EUROPEAN PATENT APPLICATION

(11) **EP 4 470 532 A1**
(43) Date of publication of application: **04.12.2024**
(21) Application number: 23759253.0
(22) Date of filing: 23.02.2023
(51) Int. Cl.: A61K 9/51, A61K 31/573, A61K 47/24, A61K 47/44, A61P 19/02

(54) **NANO PREPARATION FOR JOINT ANALGESIA, AND PREPARATION METHOD THEREFOR AND USE THEREOF**

(30) Priority: 25.02.2022 CN 202210184618
(71) Applicant: Xiangya Hospital, Central South University, Changsha, Hunan 410008 (CN)
(72) Inventor: LEI, Guanghua, Changsha, Hunan 410008 (CN); DENG, Caifeng, Changsha, Hunan 410008 (CN); ZENG, Chao, Changsha, Hunan 410008 (CN)
(74) Representative: Vesterinen, Jussi Tapio
(86) International application number: PCT/CN2023/077964
(87) International publication number: WO 2023/160631

(57) **Abstract**

The present disclosure discloses a nanopreparation for joint analgesia, including glucocorticoid and a nanocarrier, where the nanocarrier is mainly composed of phospholipid and an auxiliary agent, and the auxiliary agent includes polyoxyethylene castor oil. The nanopreparation of the present disclosure can be used for joint analgesia, has a large drug load, and can be retained in the joint cavity for at least 4 weeks. Its analgesic effect on OA is significantly better than that of a commercially available GC injection and can be maintained for at least 4 weeks. Further disclosed is a preparation method and use of the nanopreparation. The process is simple, and the particle size is less than 200 nm. Terminal sterilization can be achieved by probe ultrasound, high-pressure homogenization or sterilization through a microfiltration membrane, and the like, and the preparation cost is relatively low.

## Description

### TECHNICAL FIELD

The present disclosure belongs to the field of biomedicine, and in particular to a nanopreparation for joint analgesia, and a preparation method and use thereof.

### BACKGROUND

Osteoarthritis (OA) is an aging disease with joint pain as a main symptom, which is caused by multiple factors. It has a high disability rate and imposes a heavy burden on the country and society. Analgesia is the leading demand of OA patients at visit. Glucocorticoid (GC) is a class of steroidal small-molecule drugs with diverse pharmacological actions. It can exert an analgesic effect by inhibiting inflammatory responses and have been widely used for OA analgesia. However, GC has poor solubility, and has important problems that need to be solved urgently, such as a short duration of the analgesic effect, the analgesic effect being not significant enough, and potential safety hazards. For example, an immediate-release suspension injection of triamcinolone acetonide used clinically has an average retention time of 3.8 days in a knee joint of a patient with osteoarthritis, and the frequency of intra-articular injection is relatively high. Therefore, it is of great significance to improve the solubility of GC, reduce the administration frequency of GC, and prolong its analgesic effect on osteoarthritis.

Generally, encapsulating a drug in a nanocarrier of a liposome or polymer material can improve solubility of the drug. However, the liposome has a low drug load for GC and is prone to drug precipitation in a short period of time. Encapsulating GC in a nanocarrier made of a polymer material can also improve solubility of GC, but there are fewer polymer materials available for human injection and it is difficult to achieve long-term drug release after intra-articular injection. With the widespread application of biopolymer materials in the field of medicine, sustained-release microspheres based on the polymers have gradually become one of the research hotspots of new pharmaceutical preparations. Currently, the polymer sustained-release microspheres on the market are mainly polylactic acid-glycolic acid (PLGA) sustained-release microspheres. After GC (e.g., triamcinolone acetonide, dexamethasone, dexamethasone palmitate, etc.) is encapsulated in PLGA microspheres and intra-articularly injected, the analgesic effect of GC on OA can last for 4-12 weeks, and the retention time and analgesic time of the drug in the joint cavity are related to the particle size of the microspheres. However, when the particle size of the microspheres is greater than 25 µm, there is a risk of promoting the development of joint inflammation. Furthermore, for PLGA sustained-release microspheres, the preparation process is complex, terminal sterilization is difficult, and the lactic acid and glycolic acid produced during a degradation process may cause decrease in pH in the joint cavity, thereby promoting the development of inflammatory response.

### SUMMARY

A technical problem to be solved by the present disclosure is to overcome the shortcomings and defects mentioned in the Background above by providing a nanopreparation for joint analgesia made of glucocorticoid, phospholipid and polyoxyethylene castor oil, which can also achieve long-term sustained release of a drug (for more than 4 weeks) at the same time. However, its preparation process is simple and easy to control, and it is easy to sterilize. It not only solves the problem of poor solubility of GC, but also allows excipients as used to not cause risks such as local pH reduction.

In order to solve the aforementioned technical problems, the technical solution proposed by the present disclosure is:

a nanopreparation for joint analgesia, including glucocorticoid and a nanocarrier, wherein the nanocarrier is mainly composed of phospholipid and an auxiliary agent, and the auxiliary agent includes polyoxyethylene castor oil.

In the present disclosure, it has been found by research that when only phospholipid or only polyoxyethylene castor oil is used for encapsulating the glucocorticoid, stable nanoparticles cannot be formed. When the glucocorticoid is loaded into nanoparticles composed of phospholipid and polyoxyethylene castor oil, it has a stronger ability to inhibit the expression of macrophage inflammatory factors (as shown in FIG. 1, the intra-articular inflammation degree is closely related to joint pain); and we have unexpectedly found that the nanopreparation composed of the phospholipid and the polyoxyethylene castor oil can achieve a long-term sustained release effect in the inflammatory joints of arthritis (as shown in FIG. 2), so further, these nanoparticles loaded with the glucocorticoid can achieve long-term analgesia (as shown in FIG. 3).

In the aforementioned nanopreparation, preferably the glucocorticoid includes one or more of clobetasol propionate, diflorasone acetate, dexamethasone propionate, difluprednate, mometasone furoate, diflucortolone valerate, betamethasone butyrate propionate, fluocinolone acetonide, hydrocortisone propionate butyrate, beclomethasone propionate, deprodone propionate, betamethasone valerate, dexamethasone valerate, prednisolone valerate acetate, fluocinolone, hydrocortisone butyrate, clobetasone butyrate, alclomethasone propionate, triamcinolone acetonide, flumethasone pivalate, prednisolone, hydrocortisone, dexamethasone, dexamethasone palmitate, and triamcinolone acetonide palmitate. More preferably, the glucocorticoid is one or more of dexamethasone, dexamethasone palmitate, triamcinolone acetonide, and prednisolone.

Preferably, the phospholipid includes one or more of natural soybean phospholipid, natural egg yolk phospholipid, and synthetic phospholipid, and more preferably phospholipid E80, S100, PC98-T, and HSPC.

Preferably, the polyoxyethylene castor oil includes one or more of polyoxyethylene 35 castor oil (EL-35), polyoxyethylene 40 castor oil (EL-40), polyoxyethylene 40 hydrogenated castor oil (HEL-40), and polyoxyethylene 60 hydrogenated castor oil (HEL-60). More preferably, solid polyoxyethylene castor oil (at least one of HEL-40 and HEL-60) is used in combination with liquid polyoxyethylene castor oil (at least one of EL-35 and EL-40) to further increase the drug load.

Preferably, the auxiliary agent further includes polyethylene glycol 15-hydroxystearate (with the trade name of Kolliphor HS15), and the drug load is higher after addition of the polyethylene glycol 15-hydroxystearate.

Therefore, according to a preferred embodiment, the auxiliary agent includes polyoxyethylene 40 hydrogenated castor oil (HEL-40), polyoxyethylene 35 castor oil (EL-35) and polyethylene glycol 15-hydroxystearate. More preferably, the auxiliary agent is composed of polyoxyethylene 40 hydrogenated castor oil (HEL-40), polyoxyethylene 35 castor oil (EL-35) and polyethylene glycol 15-hydroxystearate.

According to another preferred embodiment, the auxiliary agent includes polyoxyethylene 60 hydrogenated castor oil (HEL-60), polyoxyethylene 35 castor oil (EL-35) and polyethylene glycol 15-hydroxystearate. More preferably, the auxiliary agent is composed of polyoxyethylene 60 hydrogenated castor oil (HEL-60), polyoxyethylene 35 castor oil (EL-35) and polyethylene glycol 15-hydroxystearate.

According to another preferred embodiment, the auxiliary agent includes polyoxyethylene 40 hydrogenated castor oil (HEL-40), polyoxyethylene 40 castor oil (EL-40) and polyethylene glycol 15-hydroxystearate. More preferably, the auxiliary agent is composed of polyoxyethylene 40 hydrogenated castor oil (HEL-40), polyoxyethylene 40 castor oil (EL-40) and polyethylene glycol 15-hydroxystearate.

According to another preferred embodiment, the auxiliary agent includes polyoxyethylene 60 hydrogenated castor oil (HEL-60), polyoxyethylene 40 castor oil (EL-40) and polyethylene glycol 15-hydroxystearate. More preferably, the auxiliary agent is composed of polyoxyethylene 60 hydrogenated castor oil (HEL-60), polyoxyethylene 40 castor oil (EL-40) and polyethylene glycol 15-hydroxystearate.

When the auxiliary agent is used in combination with the aforementioned three auxiliary agents, the three auxiliary agents may be present in any mass ratio.

Moreover, when polyoxyethylene 60 hydrogenated castor oil (HEL-60) and polyoxyethylene 35 castor oil (EL-35) are used in combination, or polyoxyethylene 60 hydrogenated castor oil (HEL-60) and polyethylene glycol 15-hydroxystearate are used in combination, nanoparticles with higher drug load can be prepared.

Therefore, according to a most preferred embodiment, the auxiliary agent includes polyoxyethylene 60 hydrogenated castor oil (HEL-60) and polyoxyethylene 35 castor oil (EL-35). More preferably, the auxiliary agent is composed of polyoxyethylene 60 hydrogenated castor oil (HEL-60) and polyoxyethylene 35 castor oil (EL-35).

According to another most preferred embodiment, the auxiliary agent includes polyoxyethylene 60 hydrogenated castor oil (HEL-60) and polyethylene glycol 15-hydroxystearate. More preferably, the auxiliary agent is composed of polyoxyethylene 60 hydrogenated castor oil (HEL-60) and polyethylene glycol 15-hydroxystearate.

When the auxiliary agent is a combination of the aforementioned two auxiliary agents, the two auxiliary agents may be present in any mass ratio.

Preferably, a mass ratio of the phospholipid to the auxiliary agent is (1:20)-(20: 1), preferably (1:10)-(10:1), more preferably (1:5)-(5:1), and more preferably (1:2)-(2:1). Most preferably, a mass ratio of the phospholipid to the auxiliary agent is 3:5.

Preferably, the drug load of the glucocorticoid is 0.5-20%.

Preferably, a particle size of the nanopreparation is less than 200 nm.

Preferably, a dosage form of the nanopreparation is an injection or a lyophilized powder for injection.

Based on a general inventive concept, the present disclosure further accordingly provides a method for preparing the nanopreparation for joint analgesia, including the following steps: dissolving glucocorticoid, phospholipid, and an auxiliary agent in an organic solvent, removing the organic solvent by a reduced-pressure evaporation method, adding water for stripping, and then obtaining the nanopreparation by probe ultrasound, high-pressure homogenization or extrusion through a microporous membrane.

Based on a general inventive concept, the present disclosure further accordingly provides use of the nanopreparation in the preparation of a drug for joint analgesia of osteoarthritis.

Compared with the prior art, the present disclosure has the following beneficial effects:
1. The nanopreparation of the present disclosure can be used for joint analgesia, has a large drug load, and can be retained in the joint cavity for at least 4 weeks. Its analgesic effect on OA is significantly better than that of a commercially available GC injection and can be maintained for at least 4 weeks.
2. The preparation method of the present disclosure has a simple preparation process, and the particle size of the nanopreparation is less than 200 nm. Terminal sterilization can be achieved by probe ultrasound, high-pressure homogenization or sterilization through a microfiltration membrane, and the like, and the preparation cost is relatively low.

### BRIEF DESCRIPTION OF THE DRAWINGS

To describe the technical solutions in the embodiments of the present disclosure or in the prior art more clearly, the following briefly describes the accompanying drawings required for describing the embodiments or the prior art. Apparently, the accompanying drawings in the following description show some embodiments of the present disclosure, and a person of ordinary skill in the art may still derive other drawings from these accompanying drawings without creative efforts.

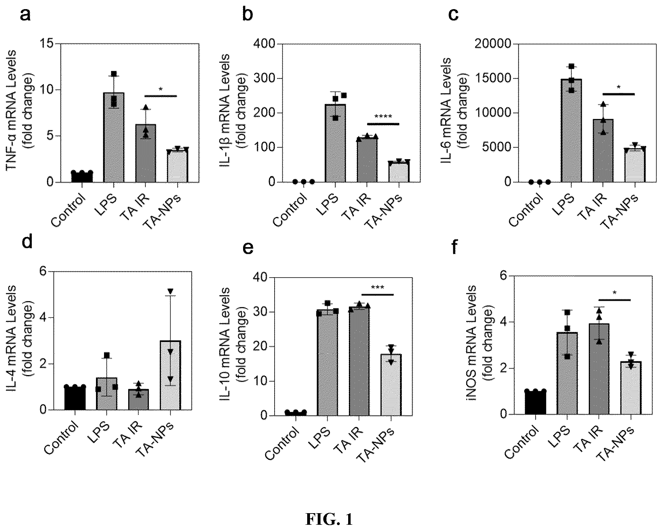

FIG. 1 shows experimental results of an *in vitro* bioactivity investigation of a nanopreparation prepared in Example 2 (24 h after drug treatment); where, a) shows the relative expression amount of a mRNA encoding a TNF-α gene in cells; b) shows the relative expression amount of a mRNA encoding a IL-1β gene in cells; c) shows the relative expression amount of a mRNA encoding a IL-6 gene in cells; d) shows the relative expression amount of a mRNA encoding a IL-4 gene in cells; e) shows the relative expression amount of a mRNA encoding a IL-10 gene in cells; and f) shows the relative expression amount of a mRNA encoding an iNOS gene in cells. *P < 0.05, ***P < 0.001, ****P < 0.0001.

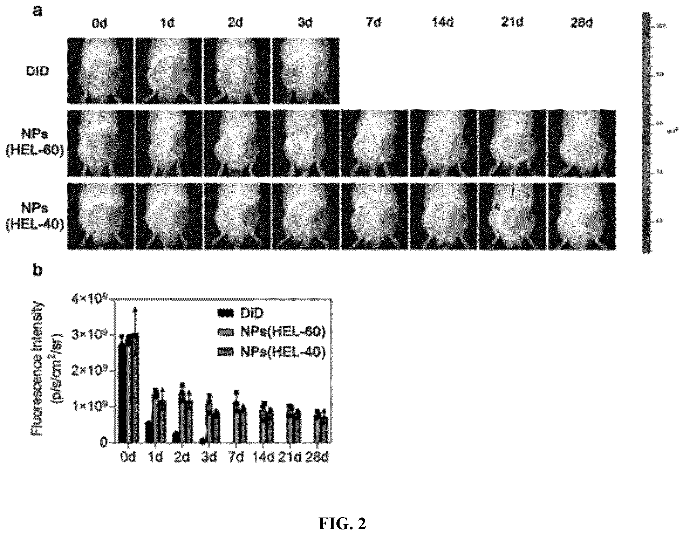

FIG. 2 shows the experimental results of *in vivo* distribution of the nanopreparations prepared in Example 1 and Example 2; where: a) is small-animal *in vivo* fluorescence imaging diagram of the distribution of NPs (HEL-40) and NPs (HEL-60) in rats of an OA model; and b) is a semi-quantitative bar graph of the distribution of NPs (HEL-40) and NPs (HEL-60) in the inflammatory joints of rats in an OA model (n = 3).

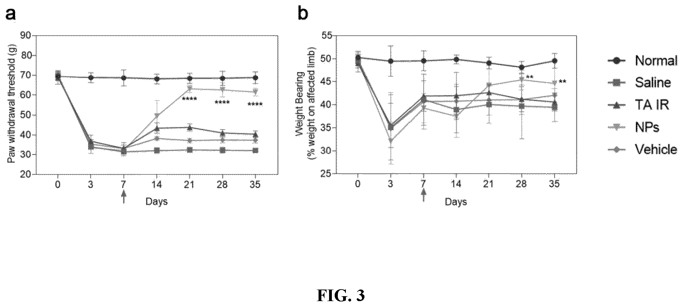

FIG. 3 shows the pharmacodynamic experimental results of the nanopreparation prepared in Example 2; where, a) is a diagram showing the change of a mechanical paw withdrawal threshold of the right hind limb of rats in an OA model (n = 7), ****P < 0.0001, compared with the commercially available injection (TA IR); and b) is a diagram showing the change of the difference in weight bearing between both feet of rats in an OA model (n = 7), **P < 0.01, compared with the commercially available injection (TAIR).

### DETAILED DESCRIPTION OF THE EMBODIMENTS

In order to facilitate understanding of the present disclosure, the present disclosure will be described more fully and in detail below with reference to the accompanying drawings and preferred embodiments of the specification, but the claimed scope of the present disclosure is not limited to the following specific examples.

Unless otherwise defined, all technical terms used hereinafter have the same meanings as commonly understood by those skilled in the art. The technical terms used herein is only for the purpose of describing specific embodiments, and is not intended to limit the claimed scope of the present disclosure.

Unless otherwise specified, various raw materials, reagents, instruments, and devices, etc. used in the present disclosure can be commercially available or can be prepared through existing methods.

The instruments and reagents used in the present disclosure are:
RV10-type rotary evaporator (IKA Group, Germany), JY92-II-type ultrasonic cell pulverizer (Ningbo Xinzhi Scientific Instrument Research Institute), EmulsiFlex-C5 high pressure homogenizer (AVESTIN, Canada), MS105DU-type electronic balance (METTLER TOLEDO, USA), Zetasizer Nano zsu3100-type laser particle size analyzer (Malvern, UK), ModulyoD freeze dryer (Thermo, USA), and Lumina 3 small-animal *in vivo* imager (Perkin Elmer, USA).

Triamcinolone acetonide (Aladdin Reagent (Shanghai) Co., Ltd.); dexamethasone (Aladdin Reagent (Shanghai) Co., Ltd.); dexamethasone palmitate (Aladdin Reagent (Shanghai) Co., Ltd.); prednisolone (Aladdin Reagent (Shanghai) Co., Ltd.); egg yolk lecithin E80 (Lipoid, Germany); soybean lecithin S100 (Lipoid, Germany); polyoxyethylene 35 castor oil (EL-35) (BASF, Germany); polyoxyethylene 40 castor oil (EL-40) (BASF, Germany); polyoxyethylene 40 hydrogenated castor oil (HEL-40) (BASF, Germany); polyoxyethylene 60 hydrogenated castor oil (HEL-60) (BASF, Germany); polyethylene glycol 15-hydroxystearate (HS15) (BASF, Germany); DiD (Biotium, USA), triamcinolone acetonide injection (Kunming Jida Pharmaceutical Co., Ltd.).

### Example 1:

A nanoparticle loaded with triamcinolone acetonide (TA) for joint analgesia was provided. The formula of the nanoparticle contained triamcinolone acetonide (TA), phospholipid E80, and HEL-40, and the dosage form was an injection. A method for preparing the nanoparticle was as follows:

4 mg of triamcinolone acetonide, 125 mg of phospholipid E80, and 70 mg of HEL-40 were dissolved in chloroform, concentrated under reduced pressure to remove the organic reagent, added with normal saline for hydration, and subjected to probe ultrasound in an ice-water bath to obtain the nanoparticle. The particle size was 133 nm.

### Example 2:

A nanoparticle loaded with triamcinolone acetonide (TA) for joint analgesia was provided. The formula of the nanoparticle contained triamcinolone acetonide (TA), phospholipid E80, and HEL-60, and the dosage form was an injection. A method for preparing the nanoparticle was as follows:
4 mg of triamcinolone acetonide, 100 mg of phospholipid E80, and 100 mg of HEL-60 were dissolved in chloroform, concentrated under reduced pressure to remove the organic reagent, added with normal saline for hydration, and subjected to probe ultrasound in an ice-water bath to obtain the nanoparticle. The particle size was 120 nm.

### Example 3:

A nanoparticle loaded with dexamethasone palmitate for joint analgesia was provided. The formula of the nanoparticle contained dexamethasone palmitate, phospholipid S100, and HEL-40, and the dosage form was an injection. A method for preparing the nanoparticle was as follows:
4 mg of dexamethasone palmitate, 125 mg of phospholipid S100, and 70 mg of HEL-40 were dissolved in chloroform, concentrated under reduced pressure to remove the organic reagent, added with normal saline for hydration, and subjected to probe ultrasound in an ice-water bath to obtain the nanoparticle. The particle size was 144 nm.

### Example 4:

A nanoparticle loaded with dexamethasone palmitate for joint analgesia was provided. The formula of the nanoparticle contained dexamethasone palmitate, phospholipid S100, and HEL-60, and the dosage form was an injection. A method for preparing the nanoparticle was as follows:
4 mg of dexamethasone palmitate, 100 mg of phospholipid S 100, and 100 mg of HEL-60 were dissolved in chloroform, concentrated under reduced pressure to remove the organic reagent, added with normal saline for hydration, and subjected to probe ultrasound in an ice-water bath to obtain the nanoparticle. The particle size was 128 nm.

### Example 5:

A nanoparticle loaded with dexamethasone for joint analgesia was provided. The formula of the nanoparticle contained dexamethasone, phospholipid S100, and HEL-60, and the dosage form was an injection. A method for preparing the nanoparticle was as follows:
2 mg of dexamethasone, 50 mg of phospholipid S100, and 10 mg of HEL-60 were dissolved in chloroform, concentrated under reduced pressure to remove the organic reagent, added with normal saline for hydration, and subjected to high-pressure homogenization to obtain the nanoparticle. The particle size was 118 nm.

### Example 6:

A nanoparticle loaded with prednisolone for joint analgesia was provided. The formula of the nanoparticle contained prednisolone, phospholipid PC98-T, and HEL-60, and the dosage form was an injection. A method for preparing the nanoparticle was as follows:
1 mg of prednisolone, 40 mg of phospholipid PC98-T, and 4 mg of HEL-60 were dissolved in a mixed solution of chloroform and ethanol, concentrated under reduced pressure to remove the organic reagent, added with normal saline for hydration, and subjected to high-pressure homogenization to obtain the nanoparticle. The particle size was 129 nm.

### Example 7:

A nanoparticle loaded with dexamethasone for joint analgesia was provided. The formula of the nanoparticle contained dexamethasone, phospholipid HSPC, and HEL-60, and the dosage form was an injection. A method for preparing the nanoparticle was as follows:
4 mg of dexamethasone, 30 mg of phospholipid HSPC, and 150 mg of HEL-60 were dissolved in chloroform, concentrated under reduced pressure to remove the organic reagent, added with normal saline for hydration, and subjected to high-pressure homogenization to obtain the nanoparticle. The particle size was 105 nm.

### Example 8:

A nanoparticle loaded with dexamethasone palmitate for joint analgesia was provided. The formula of the nanoparticle contained dexamethasone palmitate, phospholipid E80, and HEL-60, and the dosage form was an injection. A method for preparing the nanoparticle was as follows:
2 mg of dexamethasone palmitate, 15 mg of phospholipid E80, and 150 mg of HEL-60 were dissolved in chloroform, concentrated under reduced pressure to remove the organic reagent, added with normal saline for hydration, and subjected to high-pressure homogenization to obtain the nanoparticle. The particle size was 92 nm.

### Example 9:

A nanoparticle loaded with triamcinolone acetonide (TA) for joint analgesia was provided. The formula of the nanoparticle contained triamcinolone acetonide (TA), phospholipid E80, HS15, and HEL-60, and the dosage form was an injection. A method for preparing the nanoparticle was as follows:
20 mg of triamcinolone acetonide, 100 mg of phospholipid E80, 40 mg of HS15 and 60 mg of HEL-60 were dissolved in chloroform, concentrated under reduced pressure to remove the organic reagent, added with normal saline for hydration, and subjected to high-pressure homogenization to obtain the nanoparticle. The particle size was 124 nm.

### Example 10:

A nanoparticle loaded with dexamethasone for joint analgesia was provided. The formula of the nanoparticle contained dexamethasone, phospholipid E80, HS15 and EL-35, and the dosage form was an injection. A method for preparing the nanoparticle was as follows:
20 mg of dexamethasone, 100 mg of phospholipid E80, 40 mg of HS15 and 60 mg of EL-35 were dissolved in chloroform, concentrated under reduced pressure to remove the organic reagent, added with normal saline for hydration, and subjected to high-pressure homogenization to obtain the nanoparticle. The particle size was 118 nm.

### Example 11:

A nanoparticle loaded with triamcinolone acetonide (TA) for joint analgesia was provided. The formula of the nanoparticle contained triamcinolone acetonide (TA), phospholipid E80, EL-35, and HEL-40, and the dosage form was an injection. A method for preparing the nanoparticle was as follows:
20 mg of triamcinolone acetonide, 100 mg of phospholipid E80, 50 mg of EL35 and 50 mg of HEL-40 were dissolved in chloroform, concentrated under reduced pressure to remove the organic reagent, added with normal saline for hydration, and subjected to high-pressure homogenization to obtain the nanoparticle. The particle size was 136 nm.

### Example 12:

A nanoparticle loaded with dexamethasone for joint analgesia was provided. The formula of the nanoparticle contained dexamethasone, phospholipid E80, EL-35 and HEL-60, and the dosage form was an injection. A method for preparing the nanoparticle was as follows:
20 mg of dexamethasone, 100 mg of phospholipid E80, 50 mg of EL-35 and 50 mg of HEL-60 were dissolved in chloroform, concentrated under reduced pressure to remove the organic reagent, added with normal saline for hydration, and subjected to high-pressure homogenization to obtain the nanoparticle. The particle size was 132 nm.

### Example 13:

A nanoparticle loaded with triamcinolone acetonide (TA) for joint analgesia was provided. The formula of the nanoparticle contained triamcinolone acetonide (TA), phospholipid E80, and HEL-40, and the dosage form was lyophilized powder for injection. A method for preparing the nanoparticle was as follows:
4 mg of triamcinolone acetonide, 125 mg of phospholipid E80, and 70 mg of HEL-40 were dissolved in chloroform, concentrated under reduced pressure to remove the organic reagent, added with pure water for hydration, subjected to probe ultrasound in an ice-water bath, and then added with mannitol at a mass-to-volume ratio of 10%, and freeze-dried to obtain the nanoparticle.

### Example 14:

A nanoparticle loaded with dexamethasone for joint analgesia was provided. The formula of the nanoparticle contained dexamethasone palmitate, phospholipid E80, and HEL-60, and the dosage form was lyophilized powder for injection. A method for preparing the nanoparticle was as follows:
4 mg of dexamethasone, 100 mg of phospholipid E80, and 100 mg of HEL-60 were dissolved in chloroform, concentrated under reduced pressure to remove the organic reagent, added with pure water for hydration, subjected to probe ultrasound in an ice-water bath, and then added with mannitol at a mass-to-volume ratio of 10%, and freeze-dried to obtain the nanoparticle.

### Comparative Example 1:

A nanoparticle loaded with triamcinolone acetonide (TA) for joint analgesia was provided. The formula of the nanoparticle contained triamcinolone acetonide (TA), phospholipid E80, and HEL-40, and the dosage form was an injection. A method for preparing the nanoparticle was as follows:
4 mg of triamcinolone acetonide, 125 mg of phospholipid E80, and 4 mg of HEL-40 were dissolved in chloroform, concentrated under reduced pressure to remove the organic reagent, added with normal saline for hydration, and subjected to probe ultrasound in an ice-water bath to obtain the nanoparticle. The product was opaque and had precipitates.

### Comparative Example 2:

A nanoparticle loaded with triamcinolone acetonide (TA) for joint analgesia was provided. The formula of the nanoparticle contained triamcinolone acetonide (TA), phospholipid E80, and HEL-40, and the dosage form was an injection. A method for preparing the nanoparticle was as follows:
4 mg of triamcinolone acetonide, 4 mg of phospholipid E80, and 125 mg of HEL-40 were dissolved in chloroform, concentrated under reduced pressure to remove the organic reagent, added with normal saline for hydration, and subjected to probe ultrasound in an ice-water bath to obtain the nanoparticle. The particle size was 203 nm, and precipitation occurred within 30 min.

### To further demonstrate the effect of the nanopreparation of the present disclosure, the following experiments were conducted:

### 1) Effect of composition on drug load of nanopreparation

The present disclosure used the same method for preparing a nanoparticle (thin film hydration method) as that in the examples to prepare nanoparticles loaded with glucocorticoid using phospholipid or polyoxyethylene castor oil alone; at the same time, by fixing the ratio of phospholipid to polyoxyethylene castor oil, the effects of different compositions of polyoxyethylene castor oil and whether or not adding HS15 on the properties of the nanoparticles loaded with glucocorticoid prepared from the nanoparticles were investigated. As shown in Table 1, nanoparticles loaded with triamcinolone acetonide prepared by using phospholipid E80 or HEL-60 alone were very unstable or cannot encapsulate the drug well; phospholipid E80 and HEL-60 needed to be used in combination at a certain ratio to prepare nanoparticles that met the requirements of the inventor. Moreover, when HEL-60 was used in combination with EL-35 or HEL-60 was used in combination with HS15, nanoparticles with higher drug load could be prepared.

**Table 1: Effects of different compositions on the properties of nanoparticles loaded with glucocorticoid prepared from nanoparticles**

| Serial number of experimental group | 1 | 2 | 3 | 4 | 5 | 6 | 7 | 8 | 9 | 10 |
|---|---|---|---|---|---|---|---|---|---|---|
| E80 (mg) | 80 | 0 | 30 | 30 | 30 | 30 | 30 | 30 | 30 | 30 |
| HEL-60 (mg) | 0 | 80 | 50 | 0 | 50 | 25 | 25 | 30 | 30 | 20 |
| EL-35 (mg) | 0 | 0 | 0 | 0 | 0 | 25 | 0 | 10 | 20 | 30 |
| HS15 (mg) | 0 | 0 | 0 | 50 | 0 | 0 | 25 | 10 | 0 | 0 |
| Triamcinolone acetonide (mg) | 3 | 3 | 3 | 3 | 8 | 8 | 8 | 8 | 8 | 8 |
| Is there precipitates? | Yes | No (becomes turbid within 5 min) | No | Yes | Yes | No | No | No | No | No |

### 2) In vitro biological activity investigation

Mouse-derived macrophages RAW264.7 were inoculated in a 12-well cell culture plate, and stimulated with LPS for 24 h after confluence. The culture medium was replaced with a drug-containing culture medium, which were a TA solution (labeled as TA IR) and TA-loaded NPs (the concentration of TA was 4 µg/mL, the culture medium without addition of drug was used as a control, and the TA-loaded NPs were prepared according to Example 2 and labeled as TA-NPs). After incubation for 24 h, the concentrations of IL-1β, TNF-α, IL-6, IL-10, IL-4 and iNOS in the medium were determined by using real-time fluorescence quantitative PCR. Cells not treated with LPS and cells treated with LPS but not treated with TA were labeled as a "Control" group and a "LPS" group respectively.

It could be seen from FIG. 1 that, compared with other treatment methods, treatment with TA-NPs could significantly reduce the expression of genes of macrophage inflammatory factors.

### 3) OA rat model

An OA rat model was established by intra-articularly injecting sodium iodoacetate into the knee joints of SD rats. When the knee joints of the rat were swelled and the pain threshold was significantly reduced, the OA rat model was obtained.

### 4) In vivo distribution experiment

The glucocorticoid was replaced by a fluorescent marker DiD, and nanopreparations were prepared according to Example 1 and Example 2, which were respectively labeled as NPs (HEL-40) and NPs (HEL-60). A DiD solution (labeled as DiD), NPs (HEL-40) and NPs (HEL-60) were injected into the knee joint cavities of OA rats, respectively. The fluorescence intensity of the inflamed joints of the rats was investigated by a small-animal *in vivo* imager on days 0, 1, 2, 3, 7, 14, 21 and 28, respectively.

It could be seen from FIG. 2 that, compared with DiD, NPs (HEL-60) and NPs (HEL-40) could be retained in the joint cavities of the rats in the OA model for at least 28 days. These results indicated that the NPs had good sustained release in the inflamed joint cavity.

### 5) Pharmacodynamic studies

Rats in the OA model were treated with normal saline (labeled as Saline), a commercially available triamcinolone acetonide solution for injection (labeled as TA IR), TA-loaded NPs prepared in Example 2 (labeled as TA-NPs), and non-drug-loaded NPs prepared in Example 2 (labeled as Vehicle) respectively by intra-articular injection. In addition, an equal volume of normal saline was injected into the knee joint cavities of normal rats as a control (labeled as Control). The administration dosage of TA was 60 µg per knee joint, and administration was conducted on day 7 after modeling, for a total of 1 times of administration. Starting from the first day of administration, a mechanical paw withdrawal threshold and the difference in weight bearing between both feet of rats in each group were measured once a week. On day 35, behavioral detection of the rats was conducted.

As shown in FIG. 3, compared with other manners, treatment with NPs could relieve joint inflammation in OA rats more effectively. These results fully demonstrated the effectiveness of dual-cell targeted delivery of drugs to arthritic synovium for the treatment of OA. That was, the nanopreparation prepared by the present disclosure had a good application prospect in preparation of a drug for joint analgesia of osteoarthritis.

## Claims

1. A nanopreparation for joint analgesia, comprising glucocorticoid and a nanocarrier, wherein the nanocarrier is mainly composed of phospholipid and an auxiliary agent, and the auxiliary agent comprises polyoxyethylene castor oil.

2. The nanopreparation according to claim 1, wherein the glucocorticoid comprises one or more of clobetasol propionate, diflorasone acetate, dexamethasone propionate, difluprednate, mometasone furoate, diflucortolone valerate, betamethasone butyrate propionate, fluocinolone acetonide, hydrocortisone propionate butyrate, beclomethasone propionate, deprodone propionate, betamethasone valerate, dexamethasone valerate, prednisolone valerate acetate, fluocinolone, hydrocortisone butyrate, clobetasone butyrate, alclomethasone propionate, triamcinolone acetonide, flumethasone pivalate, prednisolone, hydrocortisone, dexamethasone, dexamethasone palmitate, and triamcinolone acetonide palmitate.

3. The nanopreparation according to claim 1, wherein the phospholipid comprises one or more of natural soybean phospholipid, natural egg yolk phospholipid, and synthetic phospholipid.

4. The nanopreparation according to claim 1, wherein the polyoxyethylene castor oil comprises one or more of polyoxyethylene 35 castor oil, polyoxyethylene 40 castor oil, polyoxyethylene 40 hydrogenated castor oil, and polyoxyethylene 60 hydrogenated castor oil.

5. The nanopreparation according to claim 1, wherein the auxiliary agent further comprises polyethylene glycol 15-hydroxystearate.

6. The nanopreparation according to claim 1, wherein the auxiliary agent comprises polyoxyethylene 60 hydrogenated castor oil (HEL-60), polyoxyethylene 35 castor oil (EL-35) and polyethylene glycol 15-hydroxystearate.

7. The nanopreparation according to claim 1, wherein the auxiliary agent comprises polyoxyethylene 60 hydrogenated castor oil (HEL-60) and polyoxyethylene 35 castor oil (EL-35).

8. The nanopreparation according to claim 1, wherein the auxiliary agent comprises polyoxyethylene 60 hydrogenated castor oil (HEL-60) and polyethylene glycol 15-hydroxystearate.

9. The nanopreparation according to claim 1, wherein a mass ratio of the phospholipid to the auxiliary agent is (1:20)-(20:1).

10. The nanopreparation according to claim 9, wherein a mass ratio of the phospholipid to the auxiliary agent is (1: 10)-(10: 1).

11. The nanopreparation according to any one of claims 1-10, wherein a drug load of the glucocorticoid is 0.5-20%, a particle size of the nanopreparation is less than 200 nm, and a dosage form of the nanopreparation is an injection or a lyophilized powder for injection.

12. A method for preparing the nanopreparation for joint analgesia according to any one of claims 1-11, comprising the following steps: dissolving glucocorticoid, phospholipid, and polyoxyethylene castor oil in an organic solvent, removing the organic solvent by a reduced-pressure evaporation method, adding water for stripping, and then obtaining the nanopreparation by probe ultrasound, high-pressure homogenization or extrusion through a microporous membrane.

13. Use of the nanopreparation according to any one of claims 1-11 or the nanopreparation prepared by the preparation method according to claim 12 in preparation of a drug for joint analgesia of osteoarthritis.
